(19) **Europäisches Patentamt**

**European Patent Office**

**Office européen des brevets**

(11) Publication number: **0 458 475 A2**

# (12) EUROPEAN PATENT APPLICATION

(21) Application number: 91303884.0

(22) Date of filing: 30.04.91

(51) Int. Cl.5: **C12N 9/50, C12P 21/02**

The applicant has subsequently filed a sequence listing and declared, that it includes no new matter.

(30) Priority: **21.05.90 JP 129148/90**

(43) Date of publication of application:
**27.11.91 Bulletin 91/48**

(84) Designated Contracting States:
**DE DK GB NL**

(71) Applicant: **TAKARA SHUZO CO. LTD.**
**609 Takenakacho**
**Fushimi-ku Kyoto(JP)**

(72) Inventor: **Koyama, Nbuto**
**96, Kubo, Ogura-cho**
**Uji-shi, Shiga-ken(JP)**
Inventor: **Kato, Ikunoshin**
**1-1-150, Nanryo-cho**
**Uji-shi, Kyoto-fu(JP)**
Inventor: **Matsushita, Hideyuki**
**3-4-1, Seta**
**Otsu-shi, Shiga-ken(JP)**
Inventor: **Abe, Yukichi**
**40-48, Ohmagari, Hiroshima-cho**
**Sapporo-gun, Hokkaida(JP)**
Inventor: **Ishii, Shin-Ichi**
**2-4-20, Sugano**
**Ichikawa-shi, Chiba-ken(JP)**

(74) Representative: **Marlow, Nicholas Simon et al**
**Reddie & Grose 16, Theobalds Road**
**London WC1X 8PL(GB)**

(54) **Transpeptidation reagent and method.**

(57) A transpeptidation reagent comprises an asparagine-residue specific protease. The reagent finds application in a method for producing a peptide by the transpeptidation reaction, which comprises the use of the asparagine-residue specific protease as the catalyst.

EP 0 458 475 A2

The present invention relates to enzymatic synthesis of peptides. More particularly, it relates to a reagent for transpeptidation and to a process of performing transpeptidation.

The following three methods may be mentioned as processes for obtaining a peptide having a desired sequence: (1) genetic engineering, (2) chemical synthesis, and (3) enzymatic synthesis.

Relatively long chain peptides can be obtained in large quantities by genetic engineering; desired peptides can be obtained in a short time by chemical synthesis, which is also capable of producing peptides having a variety of modified amino acids; and enzymatic synthesis, in contrast to chemical synthesis, has the advantages of not forming racemic bodies and of eliminating the need for protecting side-chain functional groups.

As practical examples of enzymatic synthesis, there may be mentioned methods using a serine carboxypeptidase, such as Carsberg's method ( Japanese Patent Publication No.500519/1981 ), Hayashi's method ( Japanese Patent Kokai No.232393/1987 ) and the method of Yokoyama, et al. ( Japanese Patent Kokai No.216497/1988 ); the method of Isowa, et al. using an endopeptidase, such as thermolysin that is a metalloprotease derived from Bacillus thermoproteolyticus [ Isowa, et al., Bull. Chem. Soc. Japan, 50, 2766 ( 1977 )]; and a method using papain [ Kullmann, W., Biochem. Biophys. Res. Commun., 91, 693 ( 1979 )]. These methods all utilize the reverse reaction of protease.

Markussen succeeded in producing human insulin by the transpeptidation reaction using trypsin ( Japanese Patent Publication No.135452/1981 ).

Genetic engineering enables the synthesis of long-chain peptides, but it is impossible to obtain a peptide that contains any amino acid residue other than the twenty kinds of amino acids coded by nucleic acid. If chemical synthesis is used to produce a peptide containing 20 or more amino acid residues, its yield is extremely low. The methods utilizing the reverse reaction of protease require the protection of amino and carboxyl terminuses in some cases. and threaten to form denatured final products because the product yield is extremely low unless an organic solvent is used for reaction. Markussen's method requires that lysine or arginine residue be present in the vicinity of the site to be transformed.

The object of the present invention is to provide a new method for enzymatically synthesizing peptides simply and at a low cost on the basis of the conventional methods described above.

Briefly, the present invention relates to a reagent for the transpeptidation reaction, which comprises asparagine-residue specific protease.

The present invention further relates to a method for producing peptides by the transpeptidation reaction, which comprises the use of an asparagine-residue specific protease as the catalyst.

As a result of intensive studies, the present inventors discovered that the protease which specifically hydrolyzes only the amide bond on the C-terminal side of L-asparagine ( called asparagine-residue specific protease ), as disclosed in Japanese Patent Application No.297450/1989 [ U.S. Ser. No.07/527729 or EP Applin. No.40305628.1 )], is capable of catalyzing transpeptidation, and accomplished the present invention based on these findings.

The asparagine-residue specific protease used in the present invention may be any protease which specifically hydrolyzes only the amide bond on the C-terminal side of L-asparagine regardless of its source or preparative method. As an example, may be mentioned the one obtained from seeds of Canavalia ensiformis ( refer to Japanese Patent Application No.297450/1989 ).

This protease is isolated from the seeds of Canavalia ensiformis as follows, as described in the specifications of the above Japanese Patent.

Seeds of Canavalia ensiformis are ground in a buffer solution, and the extract is subjected to ion-exhcange chromatography, to affinity chromatography using a column packed with paramercuribenzoic acid previously immobilized to a carrier, and to gel filtration, thus giving the asparagine-residue specific protease.

It is generally accepted that a thiol protease catalyzes hydrolysis of peptide chains through the mechanism described below. The -SH group at the enzyme active center is first acylated by a peptide or amino acid on the N-terminal side of the peptide bond to he hydrolyzed, and the enzyme is then deacylated by the nucleophilic attack of water, thus completing the reaction. If, in this case, a peptide or amino acid is present in the reaction system at a relatively high concentration, for example, 0.3M or higher, the peptide or amino acid is added to the carboxyl side of the peptide bond just cleaved by aminolysis in the step of deacylation. This reaction is called transpeptidation, and the peptide or amino acid used for aminolysis is called amine component. The probabilities that the hydrolysis and the transpeptidation will occur largely depend on the pH during reaction, the concentration of amine component, the kind of protease and other factors.

Intensive studies have lead us to find that the transpeptidation reaction takes place efficiently if an asparagine-residue specific protease is used as the catalyst, an amine component is used at a concentration of 0.3M or higher, and the reaction is carried out at a pH in the range from 6 to 8.5, preferably from 7.5 to 8.

As an example of the transpeptidation reaction performed by the use of an asparagine-residue specific protease, may be mentioned the one represented by the following general formula (I):

A-Asn-B + C → A-Asn-C + B     (I)

( wherein A stands for an amino acid, a peptide, a derivative thereof, or H; Asn shows asparagine residue; B denotes an amino acid, a peptide, a derivative thereof, or NH$_2$; and C represents an amino acid, a peptide, or a derivative thereof. The same is true of the following. ).

The above transpeptidation reaction represents a method for producing a peptide or a derivative thereof shown by the general formula (III),

A-Asn-C     (III)

in which an asparagine-residue specific protease is used as the catalyst in an aqueous solution containing a peptide or a derivative thereof having the general formula (II) ( hereinafter abbreviated as an "Asn component" ), and an amino acid, a peptide, or a derivative thereof, having the general formula (IV) ( hereinafter abbreviated as an "amine component" ),

A-Asn-B     (II)
H-C     (IV)

As an example of the transpeptidation reactions of this invention represented by the above formula (I), may be mentioned the ones shown by the following formula (V):

A-Asn-B + D-Asn-E → A-Asn-E + D-Asn-B     (V)

( wherein D stands for an amino acid, a peptide, a derivative thereof or H, and E denotes an amino acid, a peptide, a derivative thereof or NH$_2$, in which D and E are not the same as A and B, respectively ).

The amino acid derivative herein means an amino acid with, its $\alpha$-carboxyl group amidated or esterified, an amino acid added with sugar or sugar chain, an acylated $\alpha$-amino acid, or an amino acid with its hydroxyl group phosphorylated or sulfated. The peptide derviative herein means a peptide containing at least one residue of the above-mentioned amino acid derivative.

The peptide or a derivative thereof represented by the general formula (II) can be obtained by extraction from living bodies, by means of genetic engineering, or by chemical synthesis. The peptide, amino acid, or a derivative thereof represented by the general formula (IV) can also be obtained in a similar way.

The invention will be further explained as follows by referring partly to the accompanying drawings wherein: Fig. 1 is a graph showing the influence of pH on the transpeptidation, Fig. 2 is a graph showing the influence of the concentration of glycylglycine on the transpeptidation, and Fig. 3 is a graph showing the influence of reaction time on the transpeptidation.

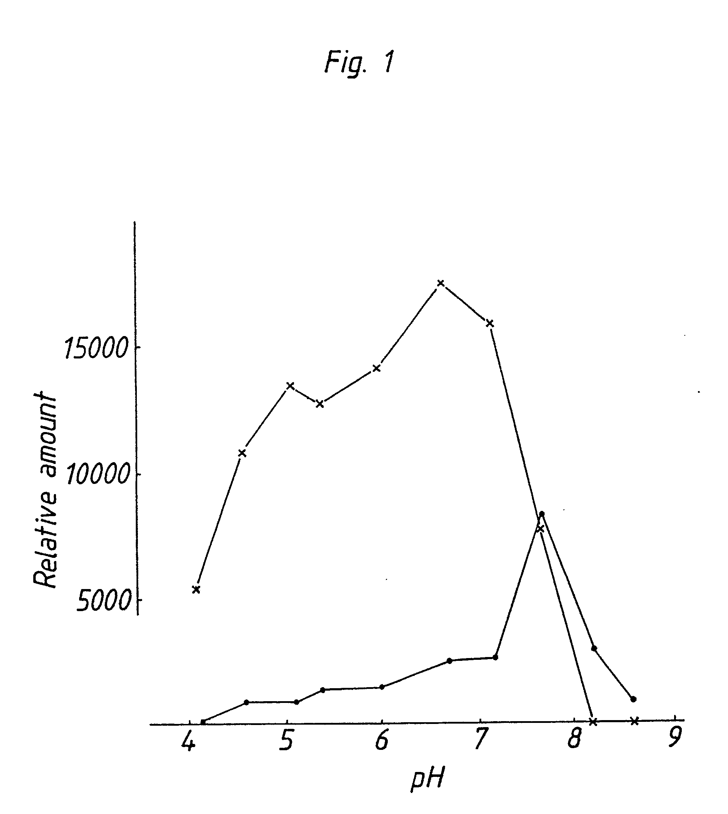

In the case of Fig. 1, DNP-Pro-Glu-Ala-Asn-NH$_2$ ( DNP: 2,4-dinitrophenyl group) ) and 0.5M Gly-Gly were used as Asn component and amine component, respectively, the reaction was carried out at 37°C for 20 minutes with the pH adjusted by using sodium hydroxide or hydrochloric acid, and the reaction mixture was analyzed by C$_{18}$ reversed-phase HPLC, indicating that the optimal pH was 7.7. The black circles show DNP-Pro-Glu-Ala-Asn-Gly-Gly formed by transpeptidation, while the X marks show DNP-Pro-Glu-Ala-Asn formed by hydrolysis ( the same is true of the following ), and relative amounts of these reaction products are plotted against the pH value.

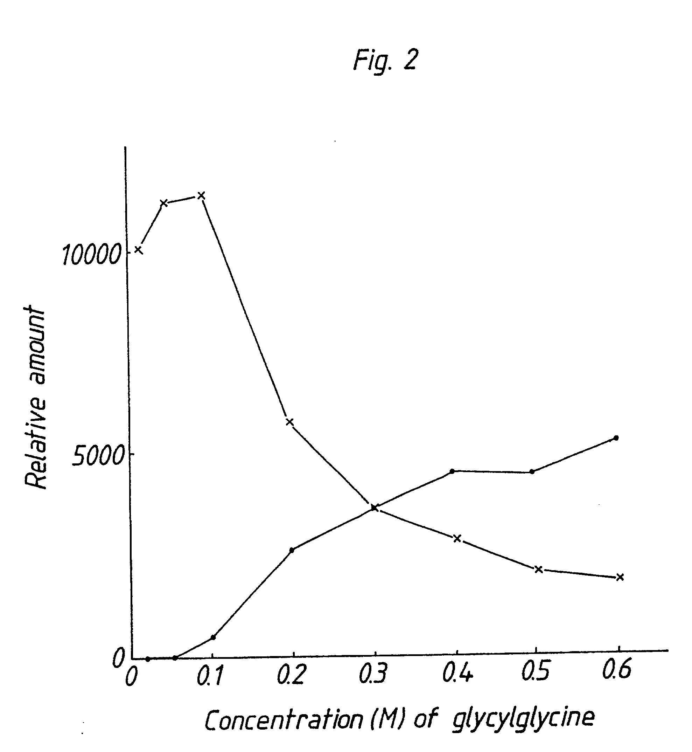

Fig. 2 shows the result of reactions carried out at pH 7.7 using various concentrations of Gly-Gly as amine component and DNP-Pro-Glu-Ala-Asn-NH$_2$ as Asn component, in which relative amounts of the reaction products are plotted against the concentration ( M ) of Gly-Gly. The transpeptidation efficiency was enhanced with increasing concentration of amino component, and the amount of its product surpassed the amount of hydrolyzate when the concentration exceeded 0.3M.

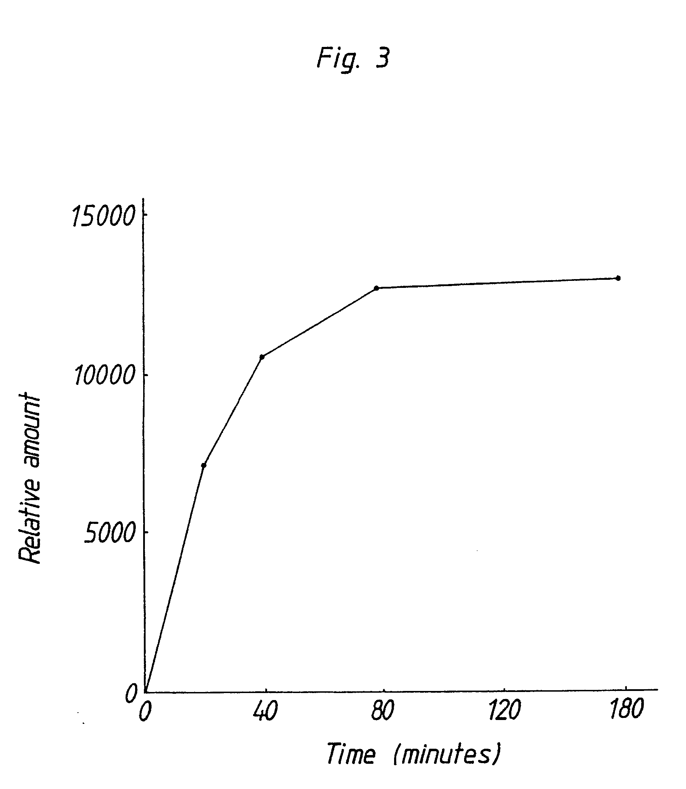

Fig. 3 shows the result of reactions carried out at pH 7.7 using 0.5M Gly-Gly as amine component and DNP-Pro-Glu-Ala-Asn-NH$_2$ as Asn component at a temperature of 37°C for various periods of time, in which the relative amount of DNP-Pro-Glu-Ala-Asn-Gly-Gly formed by transpeptidation is plotted against the reaction time ( minute ). This enzyme is stable at a pH in the range from 4.5 to 6 but is unstable at a higher pH level, and hence its inactivation terminated the reaction when the reaction time exceeded 80 minutes.

The present invention enables easy synthesis of a variety of peptides, and is favorable especially for the synthesis of physiologically active peptides with the C-terminus amidated.

The following Examples will further illustratre the invention but are not intended to limit its scope.

Example 1

First, 500 g of commercially available jack bean meal ( Sigma ) was homogenized in 20mM acetate buffer containing 1 mM DTT and 1 mM EDTA ( pH 5.0 ), the homogenate thus obtained was centrifuged, and the supernatant was dialyzed against the same buffer as above. After removing the formed precipitate by centrifugation, the supernatant was subjected to ion-exchange chromatography using a column packed with SP-Toyopearl 650M ( Tosoh Corp. ). The substance adsorbed to SP-Toyopearl 650M was eluted with 0.5M sodium chloride solution, and the active fraction was precipitated by adding ammonium sulfate to 60% saturation and dialyzed against 20mM acetate buffer containing 0.1M sodium chloride, 1 mM DTT and 1 mM EDTA ( pH 5.0 ). The dialyzate was then subjected to chromatography on paramercuribenzoic acid immobilized to Toyopearl ( Tosoh Corp. ), and the adsorbed portion was eluted with an acetate buffer containing 1M sodium chloride, 50mM DTT and 1 mM EDTA ( pH 5.0 ). The eluate was again subjected to affinity chromatography using a leucylargininal column (Organo), and the active fraction which was not adsorbed was collected. This active fraction was concentrated by the use of an ultra-filtration membrane with a cut-off molecular weight of 10,000, and the concentrate was subjected to gel filtration using Toyopearl HW-55s ( Tosoh Corp. ). The asparagine-residue specific enzyme preparation thus obtained was used in the experiments described below.

Example 2

Using 0.02mM DNP-Pro-Glu-Ala-Asn-NH$_2$ as Asn component and 0.5M Gly-Gly as amine component, transpeptidation reaction, was allowed to proceed at 37°C for 20 minutes in a solution containing 5mM DTT and 0.0048mU enzyme at various pH levels. The pH adjustment was effected by the use of 1M-HCl and 1N-NaOH. The reaction mixture was subjected to HPLC using a C$_{18}$ reversed phase column, the fraction of 350 nm absorbance peak was collected and hydrolyzed with 6N-HCl, and the hydrolyzate was identified by amino acid analysis. The result obtained is summarized in Fig. 1, which indicates that this enzyme mainly catalyzed deamidation reaction under weakly acidic conditions and catalyzed transpeptidation reaction in the region of neutrality.

Example 3

Transpeptidation was performed in much the same way as in Example 2 except that 0.5M Gly-Gly-NH$_2$ ( pH 7.7 ) was used as amine component in place of Gly-Gly. The reaction mixture was subjected to HPLC, the fractions corresponding to peaks other than those of DNP-Pro-Glu-Ala-Asn and the amide derivative thereof were collected and hydrolyzed with hydrochloric acid, and the hydrolyzate was subjected to amino acid analysis, thus demonstrating transpeptidation.

Example 4

Using 0.2mM of a peptide composed of 11 amino acid residues, His-Tyr-Ile-Asn-Leu-Ile-Thr-Arg-Gln-Arg-Tyr-NH$_2$, as Asn component and 0.5M of Gly-Gly, Gly-Gly-His, Gly-NH$_2$ or Gly-Gly-NH$_2$ as amine component, transpeptidation reaction was performed at 37°C for three hours at a pH level of 7.7, the reaction mixture was subjected to C$_{18}$ reverse-phase HPLC, and the fractions collected were analyzed to determine the amino acid sequence of each peptide, thus demonstrating transpeptidation. The molar ratios of each transpeptidation products, that is His-Tyr-Ileu-Asn-Gly-Gly, His-Tyr-Ileu-Asn-Gly-Gly-His, His-Tyr-Ileu-Asn-Gly-NH$_2$ and His-Tyr-Ileu-Asn-Gly-Gly-NH$_2$ to hydrolysis product, that is His-Tyr-Ileu-Asn were 1:1, 1:1, 7:1 and 4:1, respectively, indicating that the transpeptidation reaction had proceeded in a high yield.

As detailed above, the prsent invention provides a method for adding a peptide, amino acid, or a derivative thereof to the carboxyl side of L-asparagine residue in a peptide chain.

The present invention enables enzymatic synthesis of various peptides having physiological activiy.

## Claims

1. A transpeptidation reagent which comprises an asparagine-residue specific protease.

2. A method for producing a peptide by the transpeptidation reaction, which comprises the use of an asparagine-residue specific protease as the catalyst.

Fig. 1

Fig. 2

Fig. 3

INFORMATION FOR SEQ ID NO:1:

  (i)  SEQUENCE CHARACTERISTICS:

      (A)  LENGTH:  4 amino acid residues

      (B)  TYPE:  amino acid

      (C)  STRANDEDNESS: single

      (D)  TOPOLOGY:  linear

  (ix)  FEATURE:

      (A)  NAME/KEY: modified-site

      (B)  LOCATION: 1

      (C)  IDENTIFICATION METHOD:

      (D)  OTHER INFORMATION: /note=

"2,4-dinitrophenyl proline"

  (ix)  FEATURE:

      (A)  NAME/KEY: modified-site

      (B)  LOCATION: 4

      (C)  IDENTIFICATION METHOD:

      (D)  OTHER INFORMATION: /note= "Asn-NH$_2$"

  (xi)  SEQUENCE DESCRIPTION: SEQ ID NO:1:

Xaa Glu Ala Xaa

1

INFORMATION FOR SEQ ID NO:2:

  (i)  SEQUENCE CHARACTERISTICS:

      (A)  LENGTH:  6 amino acid residues

      (B)  TYPE:  amino acid

      (C)  STRANDEDNESS:  single

      (D)  TOPOLOGY:  linear

  (ix)  FEATURE:

      (A)  NAME/KEY: modified-site

      (B)  LOCATION: 1

      (C)  IDENTIFICATION METHOD:

5

(D)   OTHER INFORMATION: /note=

"2,4-dinitrophenyl proline"

(xi)   SEQUENCE DESCRIPTION: SEQ ID NO:2:


Xaa Glu Ala Asn Gly Gly
1                   5


INFORMATION FOR SEQ ID NO:3:

(i)   SEQUENCE CHARACTERISTICS:

(A)   LENGTH:  4 amino acid residues

(B)   TYPE:  amino acid

(C)   STRANDEDNESS:  single

(D)   TOPOLOGY:  linear

(ix)   FEATURE:

(A)   NAME/KEY: modified-site

(B)   LOCATION: 1

(C)   IDENTIFICATION METHOD:

(D)   OTHER INFORMATION: /note=

"2,4-dinitrophenyl-proline"

(xi)   SEQUENCE DESCRIPTION: SEQ ID NO:3:


Xaa Glu Ala Asn
1


INFORMATION FOR SEQ ID NO:4:

(i)   SEQUENCE CHARACTERISTICS:

(A)   LENGTH:  11 amino acid residues

(B)   TYPE:  amino acid

(C)   STRANDEDNESS:  single

(D)   TOPOLOGY:  linear

(ix)   FEATURE:

(A)   NAME/KEY: modified-site

(B)   LOCATION: 11

(C)   IDENTIFICATION METHOD:

(D) OTHER INFORMATION: /note = "Tyr-NH$_2$"

(xi) SEQUENCE DESCRIPTION: SEQ ID NO:4:


His Tyr Ile Asn Leu Ile Thr Arg Gln Arg Xaa
1                    5                    10


INFORMATION FOR SEQ ID NO:5:

(i) SEQUENCE CHARACTERISTICS:

(A) LENGTH: 6 amino acid residues

(B) TYPE: amino acid

(C) STRANDEDNESS: single

(D) TOPOLOGY: linear

(xi) SEQUENCE DESCRIPTION: SEQ ID NO:5:


His Tyr Ile Asn Gly Gly
1                    5


INFORMATION FOR SEQ ID NO:6:

(i) SEQUENCE CHARACTERISTICS:

(A) LENGTH: 7 amino acid residues

(B) TYPE: amino acid

(C) STRANDEDNESS: single

(D) TOPOLOGY: linear

(xi) SEQUENCE DESCRIPTION: SEQ ID NO:6:


His Tyr Ile Asn Gly Gly His
1                    5


INFORMATION FOR SEQ ID NO:7:

(i) SEQUENCE CHARACTERISTICS:

(A) LENGTH: 5 amino acid residues

(B) TYPE: amino acid

(C) STRANDEDNESS: single

(D) TOPOLOGY: linear


7

(ix)  FEATURE:

    (A)  NAME/KEY: modified-site

    (B)  LOCATION: 5

    (C)  IDENTIFICATION METHOD:

    (D)  OTHER INFORMATION: /note= "Gly-NH$_2$"


(xi)  SEQUENCE DESCRIPTION: SEQ ID NO:7:


His Tyr Ile Asn Xaa

                    5


(9)  INFORMATION FOR SEQ ID NO:8:

  (i)  SEQUENCE CHARACTERISTICS:

    (A)  LENGTH:  6 amino acid residues

    (B)  TYPE:  amino acid

    (C)  STRANDEDNESS:  single

    (D)  TOPOLOGY:  linear

  (ix)  FEATURE:

    (A)  NAME/KEY: modified-site

    (B)  LOCATION: 6

    (C)  IDENTIFICATION METHOD:

    (D)  OTHER INFORMATION:  /note= "Gly-NH$_2$"


(xi)  SEQUENCE DESCRIPTION: SEQ ID NO:8:


His Tyr Ile Asn Gly Xaa

1                5


INFORMATION FOR SEQ ID NO:9:

  (i)  SEQUENCE CHARACTERISTICS:

    (A)  LENGTH:  4 amino acid residues

    (B)  TYPE:  amino acid

    (C)  STRANDEDNESS:  single

    (D)  TOPOLOGY:  linear

8

(xi) SEQUENCE DESCRIPTION: SEQ ID NO: 9:

His Tyr Ile Asn
1